# EUROPEAN PATENT APPLICATION

(11) **EP 4 374 895 A1**
(43) Date of publication of application: **29.05.2024**
(21) Application number: 22306722.4
(22) Date of filing: 22.11.2022
(51) Int. Cl.: A61M 5/28, A61M 5/32

(54) **SYRINGE ASSEMBLY FOR USE IN MEDICATION DELIVERY**

(71) Applicant: Becton Dickinson France, 38800 Le Pont-de-Claix (FR)
(72) Inventor: CHANSAVANG, Albert, 38000 Grenoble (FR); CHANIOL, Henry-Gilles, 38120 Fontanil-Cornillon (FR); MILLS, Freddy, 38000 Grenoble (FR)
(74) Representative: Germain Maureau

(57) **Abstract**

A syringe assembly for use in medication delivery comprising: a syringe barrel (12) having a needle (11) at a distal end (122) of the barrel (12); a body (2) defining an enclosure, said barrel (12) extending at least partially within said enclosure; a safety shield (3) for the needle (11), longitudinally movable with respect to the body (2) between a retracted position and an activated position; a spring (4) urging said safety shield (3) towards said activated position; a first locking mechanism (31) for locking the safety shield (3) in the retracted position, wherein the first locking mechanism (31) is manually releasable by a user so as to let the safety shield (3) reach the activated position, the first locking mechanism (31) comprising a trigger button (20) on the body (2), and a first engaging area (21) on the safety shield (3); the trigger button (20) presenting a default state and a depressed state; the first locking mechanism (31) being configured such that:
∘ when the safety shield (3) is in the retracted position and the trigger button (20) is in the default state, the trigger button (20) engages the first engaging area (21);
∘ when the trigger button (20) is in the depressed state, the trigger button (20) does not engage the first engaging area (21).

## Description

### FIELD OF THE INVENTION

The present invention relates to a needle shielding system, and precisely to a syringe assembly for use in medication delivery, comprising a shield for enclosing a syringe needle after drug delivery completion.

### BACKGROUND OF THE INVENTION

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction of injection, with respect to the safety device or syringe of the invention, and the "proximal direction" is to be understood as meaning the opposite direction to said direction of injection, that is to say the direction towards the user's hand.

Pre-fillable or prefilled syringes, usually comprise a hollow tubular body or barrel forming a container for a medical product. This body comprises a distal end in the form of a longitudinal tip defining an axial passageway through which the medical product is expelled from the container. The distal end is equipped with a needle for injection of the medical product into an injection site.

It is of great importance that the patients and users are protected from any risk of needle stick injuries, particularly between the moment that injection is finished and the discarding of the drug delivery device.

Needle shielding devices can be of the so called active type where a shielding device is activated by a user after injection. An example of shielding system is Becton Dickinson Preventis^{™} needle shielding system which enables one-handed, controlled activation that automatically shields the needle. After the injection, a further pressure on the piston rod triggers the shielding as disclosed for example by EP1397170.

This solution is very satisfactory.

### SUMMARY OF THE INVENTION

In this context, it could be desirable that the activation force was completely independent from the injection movement and could be adjusted for usability purpose, for instance to allow the shielding even after a partial injection (which could be required in some cases because of the age or the weight of the patient).

According to a first aspect, the invention concerns a syringe assembly for use in medication delivery comprising:
- a syringe barrel having a needle at a distal end of the barrel;
- a body defining an enclosure, said barrel extending at least partially within said enclosure;
- a safety shield for the needle, longitudinally movable with respect to the body between a retracted position and an activated position;
- a spring urging said safety shield towards said activated position;
- a first locking mechanism for locking the safety shield in the retracted position, wherein the first locking mechanism is manually releasable by a user so as to let the safety shield reach the activated position.
- the first locking mechanism comprising a trigger button on the body, and a first engaging area on the safety shield; the trigger button presenting a default state and a depressed state;
- the first locking mechanism being configured such that
   ∘ when the safety shield is in the retracted position and the trigger button is in the default state, the trigger button engages the first engaging area;
      ∘ when the trigger button is in the depressed state, the trigger button does not engage the first engaging area.

According to some embodiments, a second locking mechanism for permanently and non-reversingly locking the safety shield in the activated position after the first locking mechanism is released.

According to some embodiments, the second locking mechanism comprises said trigger button, and a second engaging area on the safety shield.

According to some embodiments, the first locking mechanism is configured such that when the safety shield is in the activated position and the trigger button is in the default state, the trigger button engages the second engaging area.

According to some embodiments, the first locking mechanism is further configured such that when the safety shield is in the activated position and the trigger button is in the depressed state, the trigger button still engages the second engaging area.

According to some embodiments, the first engaging area is a radial protrusion at a distal part of the safety shield, and the second engaging area is a radial protrusion at a proximal part of the safety shield.

According to some embodiments, the trigger button comprises a jaw part for engaging the first and/or second engaging area through the body, and a pressure part for being pressed by the user so as to switch the trigger button from the default state to the depressed state.

According to some embodiments, the trigger button is configured such that when the pressure part is pressed, the jaw part is lifted.

According to some embodiments, the trigger button is configured such that it is urged toward the default state.

According to some embodiments, the needle is covered by the safety shield in the activated position and the needle is not covered by the safety shield in the retracted position.

According to some embodiments, each of the body and the safety shield presents at least one window such that, when the safety shield is in the retracted position, the barrel is visible through said windows.

According to some embodiments, the syringe assembly comprises a prefilled syringe comprising said barrel, said needle, a stopper and a plunger rod.

According to a second aspect, the invention concerns a shielding system for a syringe for use in medication delivery comprising a barrel and a needle at a distal end of the barrel, said shielding system comprising:
- a body defining an enclosure, said barrel extending at least partially within said enclosure;
- a safety shield for the needle, longitudinally movable with respect to the body between a retracted position and an activated position;
- a spring urging said safety shield towards said activated position;
- a first locking mechanism for locking the safety shield in the retracted position, wherein the first locking mechanism is manually releasable by a user so as to let the safety shield reach the activated position;
- the first locking mechanism comprising a trigger button on the body, and a first engaging area on the safety shield; the trigger button presenting a default state and a depressed state;
- the first locking mechanism being configured such that
   ∘ when the safety shield is in the retracted position and the trigger button is in the default state, the trigger button engages the first engaging area;
   ∘ when the trigger button is in the depressed state, the trigger button does not engage the first engaging area.

According to a third aspect, the invention concerns a method for operating a syringe assembly or a shielding system, comprising a step of triggering release of the trigger button by the first locking mechanism so that the spring moves the safety shield from the retracted position towards the activated position.

### BRIEF DESCRIPTION OF THE DRAWINGS

The invention and the advantages arising therefrom will clearly emerge from the detailed description that is given below with reference to the appended drawings as follows:
FIG. 1 is a top perspective view of an embodiment of a syringe assembly according to the present invention, with a needle safety shield in a retracted position;
FIG. 2 is a top perspective view of the syringe assembly shown in FIG. 1, with the needle safety shield in an activated position;
FIG. 3 is a top perspective view of another embodiment of a syringe assembly according to the present invention, with a needle safety shield in the activated position.

### DETAILED DESCRIPTION OF EXEMPLARY EMBODIMENTS

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. Further, terms such as distal, proximal, up, down, bottom, and top are relative, and are to aid illustration, but are not limiting. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure.

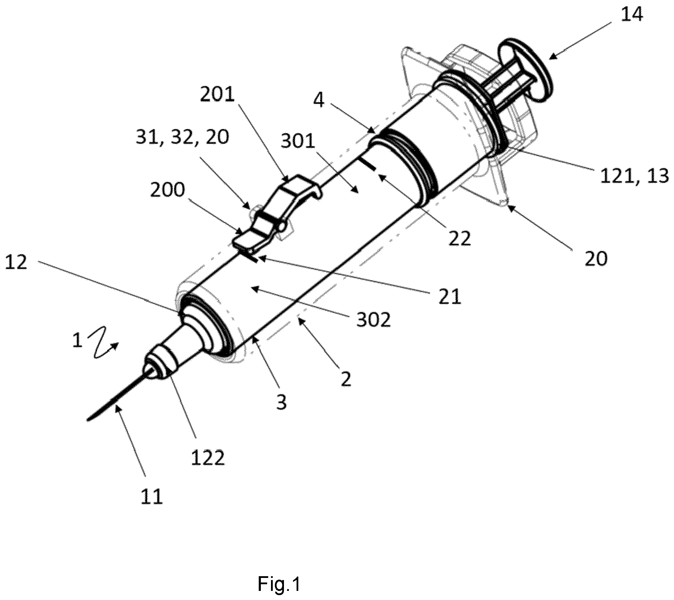

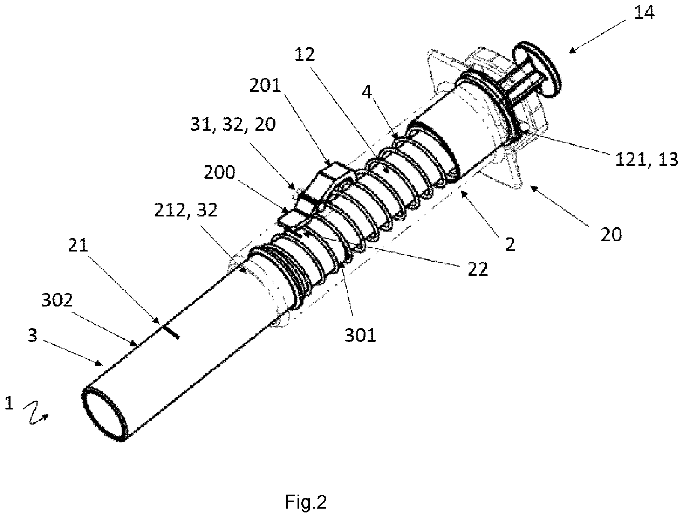

Figures 1 and 2 illustrate perspective views of a syringe assembly 1 in accordance with an embodiment of the present invention, respectively in a first state and a second state.

Said syringe assembly 1 can be use in medication delivery (in particular hypodermic injection) and preferably comprises a complete syringe 1 (in particular a pre-fillable syringe, which typically contains a single dose of medication such as such as 0.3 ml, 0.5 ml or 1.0 ml), coupled to a body 2 which enables the shielding function. Syringes are typically comprised of a barrel 12, having a proximal end 121 and a distal end 122 with respect to said longitudinal axis; a needle 11 (or other piercing or connecting element such as a cannula) secured to the distal end 121 of the barrel 12; a stopper slidably positioned within the barrel 12; and a plunger rod 14 engageable with the stopper. The barrel 12 defines an inner fluid chamber. Forward movement (along said longitudinal axis) of the plunger rod 14 moves the stopper from the proximal end 121 towards the distal end 122 which expels fluid from the barrel 12 through the needle 11. Rearward movement in the proximal direction of the plunger rod 14 will draw fluid into the barrel 12. Glass barrels are commonly used in prefillable or prefilled syringes, but plastic barrel are also known.

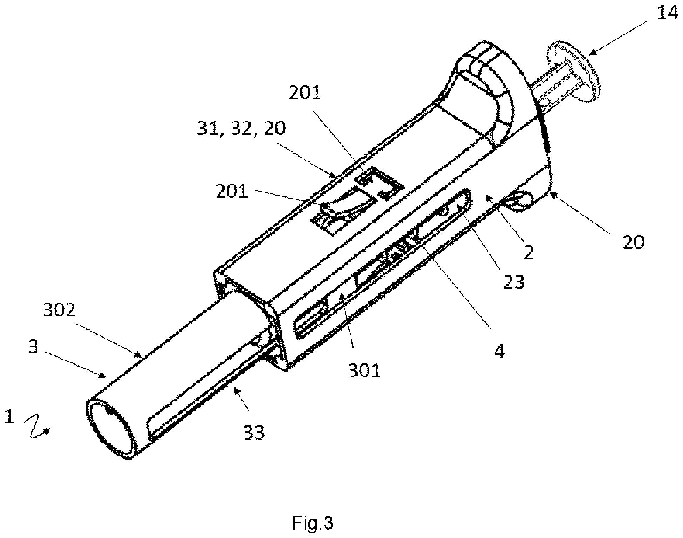

The syringe barrel 12 is coupled to a body 2 shown in dotted lines on Figure 1 and Figure 2. The body 2 can be a tubular body, and preferably an elongate, generally cylindrical piece, in particular made of plastic (preferably a semi-rigid plastic material such as polypropylene), which defines a generally cylindrical enclosure which advantageously shares the same longitudinal axis with the barrel 12. Other shapes can be contemplated. Figure 3 depicts a syringe assembly 1 having a substantially parallelepipedic body 2.

The body 2 has proximal and distal open ends which provide access to the enclosure. A finger flange 20 extends radially outwardly from the body 2 near the proximal open end thereof. The finger flange 20 and body 2 can be designed for easy handling when an injection is performed so that an injection can be carried out using a single hand. The syringe assembly also comprises a safety shield 3, longitudinally movable with respect to the body 2 - and consequently movable with respect to the barrel 12 - between a retracted position and an activated position, and a spring 4 configured for moving the shield 3. Preferably, the shield 3 is movable according to the same longitudinal axis as that of the barrel 12 and/or the body 2. Like the body 2, the shield 3 also has preferably an elongate, generally cylindrical piece, in particular made of plastic such as for example a semi-rigid plastic material such as polypropylene. The spring 4 is preferably a metal coil spring, but other energy releasing element can be contemplated, such as deformable blades. The length of the shield 3 is at least the length of the needle 11.

The syringe barrel 12 extends at least partially, and preferably substantially entirely, within the enclosure of the body 2. The barrel 12 can be slidably engaged within the body 2, and thus the syringe barrel 12 may include a radially outwardly extending flange for coupling onto an existing pre-fillable or prefilled syringe to the body 2. In other embodiments, the body 2 can be permanently secured on the barrel 12.

In any case, the needle safety shield 3 and the spring 4 can be placed inside the body 2. An annular clearance is preferably kept in the enclosure between the body 2 and the barrel 12 for housing the shield 3 and the spring 4. The safety shield 3 and the spring 4 are thus preferably configured and sized in diameter to be positioned within the body 2, and to fit over the barrel 12.

In some embodiments, the needle 11 is covered by the safety shield 3 in the activated position and the needle 11 is un-covered by the safety shield 3 in the retracted position. By covering the needle 11 is meant enclosing the needle tip such that it is not exposed, preventing any risk of injury.

In other words, in the retracted position the shield 3 is partially, and preferably entirely, engaged within the enclosure of the body 2, such that the needle 11 is exposed for use, while in the activated position (or "extended" position) the shield 3 axially protrudes from the body 2 toward the distal end, hence sheathing the needle 11.

It is referred to as "first state" the state of the present syringe assembly wherein the shield 3 is in the retracted state (see figure 1), and it is referred to as "second state" the state of the present syringe assembly wherein the shield 3 is in the activated state (see figure 2).

The spring 4 is urging said safety shield 3 towards said activated position. To this end, the body 2 can include an end shoulder (close to its proximal open end) and in the retracted position, the spring 4 is compressed between the shield 3 and said end shoulder. Therefore, the spring 4 pushes the shield 3 in the longitudinal direction: "Relaxation" of the spring 4 causes a longitudinal movement of its distal extremity and then a longitudinal movement of the shield 3 so as to proximally extend from the distal end of the body 2 and then reach the activated position. In other embodiments not shown, the proximal extremity of the spring 4 may directly be secured to the body 2.

In some embodiments, the syringe assembly 1 includes a first locking mechanism 31 for locking the safety shield 3 in the retracted position. Indeed, without said first locking mechanism 31, the shield 3 could not stay in the retracted position.. In other words, said first locking mechanism 31 holds in place the shield 3 and/or the spring 4, and thus prevents the spring 4 from longitudinally moving the shield 3 towards the activated position. The force of the spring 4 is insufficient by itself to cause disengagement of the shield 3.

However, the first locking mechanism 31 is manually releasable by a user so as to let the safety shield 3 reaches the activated position. In other words, the user can, by an action (see below) trigger the deactivation of the first locking mechanism 31 such that it stops restricting movement of the shield 3 and/or the spring 4. Thanks to the spring 4, the shield 3 switches from the retracted position to the activated position. It is referred to as "activation of the shield" the manual release of the first locking mechanism 31, hence the word "activated" state of the shield 3 when extended.

The shield 3 is naturally held in the activated position by the spring 4, but a sufficient force could move the safety shield 3 back to the retracted position and then making apparent the needle 11. In some embodiments, the present syringe assembly advantageously comprises a second locking mechanism 32 for locking the safety shield 3 in the activated position after the first locking mechanism 31 is released. A difference with the first locking mechanism 31 is that the second locking mechanism 32 is not manually releasable, even unintentionally by a user (or at least significantly more difficult to release): the needle 11 cannot become exposed again, hence complete safety is guaranteed.

The safety shield 3 is initially held by the first locking mechanism 31 in the retracted position, then, after medication delivery completion, the user releases it and the safety shield 3 longitudinally moves up to engage the second locking mechanism 32, which prevents it from switching back to the restricted position.

The operation of the body 2, the shield 3 and the locking mechanisms 31, 32, is independent from the injection movement. For instance, while figure 2 represents the plunger rod 14 completely pulled down, i.e. the medication fully delivered, it is in practice possible to deploy the shield 3 regardless of the position of the plunger rod 14.

In particular, as depicted by figures 1 and 2:
- the body 2 includes at least one button 20, i.e. an organ presenting a default state, or "lifted" state, and a depressed state. In other words, the user can press the button 20, which lowers it (the button 20 is thus switched from the default state to the depressed state). As it will be explained, the trigger button 20 is part of the first locking mechanism 31, and preferably part of both the first and second locking mechanisms 31, 32.
- the safety shield 3 includes at least a first engaging area 21, and preferably at least a second engaging area 22. Said first and second engaging areas 21, 22 are to interact with the trigger button 20, and can take many forms, for instance protrusions, slots, high friction area, etc., see below. In any case, the shield 3 and the engaging areas 21, 22 are integral, and therefore they move together. The first engaging area 21 is at a distal part 302 of the safety shield 3 and it faces the trigger button 20 when the safety shield 3 is in the retracted position (see figure 1). The second engaging area 22 is at a proximal part 301 of the safety shield 3 and it faces the trigger button 20 when the safety shield is in the activated position (see figure 2).

In the embodiment of figures 1 and 2, the trigger button 20 is mounted on the side of the body 2, and is slightly mobile in rotation with respect to said body 2, according to a tangential axis, when pressed. In order words, the trigger button 20 and the body 2 are two distinct parts, fixed together. It can be noted that when the button 2 is "outside" the body, there may be an opening in the body 2 for the button 20 to reach the shield 3 through the body 2.

According to the alternative embodiment of figure 3, the body 3 and the trigger button 20 are integral, i.e. the button 20 is directly cut into the side of the body 3. Therefore, the depressed state is actually a state wherein the body 2 is deflected by the pressure of the user finger.

The first locking mechanism 31 is configured (i.e. the button 20 and at least the first engaging area 21 are respectively located) such that:
∘ when the safety shield 3 is in the retracted position and the trigger button 20 is in the default state, the trigger button 20 engages the first engaging area 21, i.e. the trigger button 20 holds the first engaging area 21 and thus prevents the shield 3 from moving, despite the spring 4 pushing it;
∘ when the trigger button 20 is in the depressed state (regardless of the position of the safety shield 3), the trigger button 20 does not engage the first engaging area 21, i.e. it is not held anymore.

Similarly, the second locking mechanism 32 is configured such that:
∘ when the safety shield 3 is in the activated position and the trigger button 20 is in the default state, the trigger button 20 engages the second engaging area 22; and
∘ when the safety shield 3 is in the activated position and when the trigger button 20 is in the depressed state, the trigger button 20 still engages the second engaging area 22, which can be obtained in particular by having a second engaging area 22 different from the first engaging area 21 (for instance the second engaging area 22 may radially protrudes more than the first engaging area 21 if both are radial protrusions).

The combination of the button 20 and the engaging areas 21, 22 allows simple yet very reliable locking mechanisms.

Preferably the trigger button 20 is configured such that it is urged toward the default state. In other words, the default state is a stable state, and force is required to switch it from the default state to the depressed state. Inversely, in absence of said force, the button 20 automatically switches back to the default state.

In some embodiments, the trigger button 20 comprises a jaw part 200 for engaging the first and/or second engaging area 21, 22 through the body 2 (as explained, there may be a small opening in the body 2 for the jaw 200 to reach the shield 3, and the first and second engaging area 21, 22 are typically protrusions), and a pressure part 201 for being pressed by the user so as to switch the trigger button 20 from the default state to the depressed state, see in particular figure 1. In this embodiement, pressing the pressure part 201 causes rotation of the whole button around the axis, and the jaw part 200 is lifted, and the first or second engaging area 21, 22 is not held anymore. In other words, in the default state, the jaw part 200 forms a firm contact with the first or second engaging area, hence the engagement, while in the depressed state the jaw part 200 is spaced from the first or second engaging area, hence the disengagement.

In some embodiments, there may be a biasing component such a elastic blade, a torsion spring or a spring under the pressure part 201 as a means for urging the button 20 toward the default state (pressing the pressure part 201 further compress this spring). Therefore, the biasing component pushes the pressure part 201 in the radial direction, towards the default state, which causes the jaw part 200 to further squeeze the shield 3 (the first engaging area 21 in the retracted position).

In the embodiement of figure 3, there is no axis of rotation but still a jaw part 200 and a pressure part 201. Pressing the pressure part 201 locally deflects the body 2, which still results in a lift of the jaw part 200. In this embodiement, the trigger button 20 is configured such that it is urged toward the default state.

Note that any alternative architecture is possible, as long as the trigger button 20 engages the first engaging area 21 when the safety shield 3 is in the retracted position and the trigger button 20 is in the default state, and the trigger button 20 does not engage the first engaging area 21 when the trigger button 20 is in the depressed state.

As explained, the first locking mechanism 31 is manually releasable by the user, and/or the second locking mechanism 32 is not manually releasable by a user.

Indeed, when the safety shield 3 is in the retracted position, switching the trigger button 20 from the default state to the depressed state (i.e. pressing it) directly causes the trigger button 20 to disengage the first engaging area 21 (the jaw part 200 is lifted), and the safety shield 3 become free to move from the retracted position to the activated position under the effect of the spring 4.

This causes the trigger button 20 to engage this time the second engaging area 22. Indeed, because of the displacement of the shield 3, the second engaging area 22 is now facing the button 20, hence the engagement. This maintains the shield 3 in the retracted position.

When the safety shield 3 is in the activated position, switching the trigger button 20 from the default state to the depressed state (i.e. pressing it) does not cause the trigger button 20 to disengage the first engaging area 21 (if for instance the second engaging area 22 is thicker than the first second engaging area 22). Hence the second locking mechanism 32 is not manually releasable by a user and once locked is largely non-reversible.

Even if the first and second engaging areas 21, 22 were sensibly identical, unlocking the second locking mechanism 32 would require simultaneously pressing the trigger button 20 and pulling the spring, which is very difficult to the user and cannot happen by accident.

In another embodiment, in addition or alternatively, there is a third engaging area (not represented) on the safety shield 3, which is another radial protrusion, which engages the pressure part 201 of the button 20 (and not the jaw part 200) when the safety shield 3 is in the activated position. In other words, this third engaging area 23 protrudes below the pressure part 201 (if necessary through another opening in the body 2) and prevents it from being pressed. Therefore, when in the activated position, the button 20 cannot be pressed anymore, and thus the second engaging area 22 cannot be disengaged anymore.

In some embodiments, each of the body 2 and the safety shield 3 may presents at least one window 24, 34 (typically rectangular windows) such that, when the safety shield 3 is in the retracted position, the barrel 12 is visible through said windows 23, 33, as depicted by figure 2. Any marking or labelling applied on the barrel 12 and the drug contained in the barrel 12 can be visible to a user prior to injection. Preferably, the windows 23, 33 sensibly coincidate when the shield 3 is in the retracted position.

The terminology used herein is for the purpose of describing particular aspects only and is not intended to be limiting of the disclosure. As used herein, the singular forms "a," "an," and "the" are intended to include the plural forms as well, unless the context clearly indicates otherwise. As used herein, the term "and/or" includes any and all combinations of one or more of the associated listed items. It will be further understood that the terms "comprises," "comprising," "includes," and/or "including" when used herein specify the presence of stated features, integers, steps, operations, elements, and/or components, but do not preclude the presence or addition of one or more other features, integers, steps, operations, elements, components, and/or groups thereof.

It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure.

It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present.

Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein.

It is to be understood that the present disclosure is not limited to the aspects described above and illustrated in the drawings; rather, the skilled person will recognize that changes and modifications may be made within the scope of the appended claims.

## Claims

1. A syringe assembly for use in medication delivery comprising:
- a syringe barrel (12) having a needle (11) at a distal end (122) of the barrel (12);
- a body (2) defining an enclosure, said barrel (12) extending at least partially within said enclosure;
- a safety shield (3) for the needle (11), longitudinally movable with respect to the body (2) between a retracted position and an activated position;
- a spring (4) urging said safety shield (3) towards said activated position;
- a first locking mechanism (31) for locking the safety shield (3) in the retracted position, wherein the first locking mechanism (31) is manually releasable by a user so as to let the safety shield (3) reach the activated position.
- the first locking mechanism (31) comprising a trigger button (20) on the body (2), and a first engaging area (21) on the safety shield (3); the trigger button (20) presenting a default state and a depressed state;
- the first locking mechanism (31) being configured such that
∘ when the safety shield (3) is in the retracted position and the trigger button (20) is in the default state, the trigger button (20) engages the first engaging area (21);
∘ when the trigger button (20) is in the depressed state, the trigger button (20) does not engage the first engaging area (21).

2. The syringe assembly of claim 1, comprising a second locking mechanism (32) for permanently and non-reversingly locking the safety shield (3) in the activated position after the first locking mechanism (31) is released.

3. The syringe assembly of claim 2, wherein the second locking mechanism (32) comprises said trigger button (20), and a second engaging area (22) on the safety shield (3).

4. The syringe assembly of claim 3, wherein the first locking mechanism (31) is configured such that when the safety shield (3) is in the activated position and the trigger button (20) is in the default state, the trigger button (20) engages the second engaging area (22).

5. The syringe assembly of claim 4, wherein the first locking mechanism (31) is further configured such that when the safety shield (3) is in the activated position and the trigger button (20) is in the depressed state, the trigger button (20) still engages the second engaging area (22).

6. The syringe assembly of any one of claims 3 to 5, wherein the first engaging area (21) is a radial protrusion at a distal part (302) of the safety shield (3), and the second engaging area (22) is a radial protrusion at a proximal part (301) of the safety shield (3).

7. The syringe assembly of claim 6, wherein the trigger button (20) comprises a jaw part (200) for engaging the first and/or second engaging area (21, 22) through the body (2), and a pressure part (201) for being pressed by the user so as to switch the trigger button (20) from the default state to the depressed state.

8. The syringe assembly of claim 7, wherein the trigger button (20) is configured such that when the pressure part (201) is pressed, the jaw part (200) is lifted.

9. The syringe assembly of any one of claims 1 to 8, wherein the trigger button (20) is configured such that it is urged toward the default state.

10. The syringe assembly of any one of claim 1 to 9, wherein the needle (11) is covered by the safety shield (3) in the activated position and the needle (11) is not covered by the safety shield (3) in the retracted position.

11. The syringe assembly of any one of claims 1 to 10, wherein each of the body (2) and the safety shield (3) presents at least one window (24, 34) such that, when the safety shield (3) is in the retracted position, the barrel (12) is visible through said windows (24, 34).

12. The syringe assembly of any one of claims 1 to 11, comprising a prefilled syringe (1) comprising said barrel (12), said needle (11), a stopper (13) and a plunger rod (14).

13. A shielding system for a syringe (1) for use in medication delivery comprising a barrel (12) and a needle (11) at a distal end (122) of the barrel (12), said shielding system comprising:
- a body (2) defining an enclosure, said barrel (12) extending at least partially within said enclosure;
- a safety shield (3) for the needle (11), longitudinally movable with respect to the body (2) between a retracted position and an activated position;
- a spring (4) urging said safety shield (3) towards said activated position;
- a first locking mechanism (31) for locking the safety shield (3) in the retracted position, wherein the first locking mechanism (31) is manually releasable by a user so as to let the safety shield (3) reach the activated position;
- the first locking mechanism (31) comprising a trigger button (20) on the body (2), and a first engaging area (21) on the safety shield (3); the trigger button (20) presenting a default state and a depressed state;
- the first locking mechanism (31) being configured such that
∘ when the safety shield (3) is in the retracted position and the trigger button (20) is in the default state, the trigger button (20) engages the first engaging area (21);
∘ when the trigger button (20) is in the depressed state, the trigger button (20) does not engage the first engaging area (21).

14. A method for operating a syringe assembly of any one of claims 1 to 12 or a shielding system of claim 13, comprising a step of triggering release of the trigger button (20) by the first locking mechanism (31) so that the spring (4) moves the safety shield (3) from the retracted position towards the activated position.
